# EUROPÄISCHE PATENTSCHRIFT

(11) **EP 1 328 238 B1**
(45) Veröffentlichungstag und Bekanntmachung des Hinweises auf die Patenterteilung: **19.11.2008**
(21) Anmeldenummer: 01983545.3
(22) Anmeldetag: 11.10.2001
(51) Int. Cl.: A61K 6/10, A61C 9/00

(54) **VORRICHTUNG ZUR BESTIMMUNG DES ENDES DER VERARBEITUNGSZEIT VON HÄRTBAREN DENTALMASSEN**
DEVICE FOR DETERMINING THE END OF THE PROCESSING TIME FOR HARDENING DENTAL MASSES
DISPOSITIF POUR DETERMINER LA FIN DU TEMPS DE TRAITEMENT DE MATERIAUX DENTAIRES DURCISSABLES

(30) Priorität: 23.10.2000 DE 10052548
(43) Veröffentlichungstag der Anmeldung: 23.07.2003
(73) Patentinhaber: 3M ESPE AG, 82229 Seefeld (DE)
(72) Erfinder: BRANDHORST, Gerd, 86899 Landsberg (DE); NIRSCHL, Hermann, 82229 Seefeld (DE); PEUKER, Marc, 86938 Schondorf (DE); WAGNER, Ingo, 82237 Wörthsee (DE)
(74) Vertreter: Brem, Roland
(86) Internationale Anmeldenummer: PCT/EP2001/011769
(87) Internationale Veröffentlichungsnummer: WO 2002/034209

(56) Entgegenhaltungen:
- WO-A-98/43727
- DE-A- 3 919 534
- DE-A- 19 741 674
- DE-C- 19 903 753
- DE-U- 29 906 343
- US-A- 5 017 790
- US-A- 5 063 255

## Beschreibung

Die Erfindung betrifft eine Vorrichtung und ein Verfahren zur Bestimmung des Endes der Verarbeitungszeit von härtbaren Dentalmassen, insbesondere von dentalen Abformmassen.

Zur Verarbeitung und Anwendung von Abformmassen im Dentalbereich werden üblicherweise in den Gebrauchsanweisungen entsprechende Zeiten angegeben, wie die Abformmasse zu handhaben ist und wann sie aus dem Mund des Patienten entnommen werden soll.

Die angegebenen Zeiten für Verarbeitung und Abbindeverhalten der Dentalmassen; sind jedoch in der zahnärztlichen Praxis verschiedenen Störgrößen unterworfen, wie die aktuelle Mund- und Raumtemperatur, eingebrachte Mischenergie oder die Zeit, die zum Mischen aufgewandt wurde.

Denkbar ist es, in die polymerisierbaren Massen Indikatoren einzuarbeiten, die während der Polymerisation freigesetzt werden und den Reaktionsfortgang beispielsweise durch Änderung der Farbintensität anzeigen. Ein solcher Versuch ist in der WO-96/00560 beschrieben.

Nachteilig daran ist, dass die beschriebenen Massen eine weitere Komponente enthalten, die sich negativ auf die gewünschten Eigenschaften auswirken kann. Außerdem liefert die sich über die Zeit verändernde Farbe kein eindeutig definierbares Signal über den Abbindevorgang und erfordert zudem eine ständige optische Kontrolle.

Die DE 29 906 343 U1 versucht das Problem durch Bereitstellung eines Gerätes zum Ausgeben von Mehrkomponentenmassen zu lösen, das über eine Zeituhr zur Angabe einer für die Verarbeitung relevanten Zeit ausgerüstet ist.

Das beschriebene Gerät hat allerdings den Nachteil, dass die gemischte Masse ebenfalls den genannten äußeren Einflüssen unterliegt und somit kein eindeutiger Hinweis gegeben werden kann, wann der Abbindevorgang einsetzt.

Bekannt sind ferner industriell einsetzbare Messmischer zur Konsistenzprüfung von Zement-Wasser-Mischungen, die bei der Bereitung von Beton zum Einsatz kommen (DE 199 03 753 C1).

Eine Zusatzvorrichtung zur Drehmomentmessung für Rührgeräte, insbesondere für Kleinfermenter, ist in der DE 28 50 486 A1 beschrieben.

In der DE 39 19 534 A1 wird ein Verfahren und eine Vorrichtung zur Vorbereitung von Knochenzement offenbart. Dabei taucht ein Propeller in ein Behältnis ein, in dem der Knochenzement zubereitet werden soll. Die Zubereitung des Knochenzements erfolgt prozessgesteuert.

Keine der aus dem Stand der Technik bekannten Vorrichtungen eignet sich zur Bestimmung der Änderung von rheologischen Eigenschaften von verhältnismäßig rasch erhärtenden Dentalmassen, insbesondere dentalen Abformmassen, die in kurzen Abständen hintereinander portionsweise zubereitet und zur Verfügung gestellt werden müssen.

Es ist somit eine Aufgabe der vorliegenden Erfindung, eine Vorrichtung bzw. ein Verfahren bereitzustellen, die bzw. das die oben angesprochenen Probleme löst und den Anwender über den Fortgang des Aushärtens von erhärtenden Dentalmassen, insbesondere dentalen Abformmassen, informiert.

Diese Aufgabe wird durch eine Vorrichtung und ein Verfahren gelöst, wie sie in den Ansprüchen beschrieben sind.

Der Begriff Abbindeverlauf beschreibt allgemein die während des Erhärtens oder Abbinden von härtbaren Dentalmassen auftretenden Änderungen von rheologischen Eigenschaften. Eng damit verbunden ist die Bestimmung des Endes der Verarbeitungszeit.

Unter dem Ausdruck Verarbeitungszeit ist die Zeit zu verstehen, während der die gemischte Masse noch bestimmungsgemäß angewandt werden kann. Bei dentalen Abformmassen handelt es sich um die Zeit, bis zu der die Abformmasse verhältnismäßig problemlos vom abzuformenden Gegenstand abgehoben werden kann, ohne dass in nennenswertem Umfang die Genauigkeit der Abformung beeinträchtigt wird. Üblicherweise ist dies der Zeitpunkt, bei dem die Masse vom pastösen, plastischen Zustand in einen zähelastischen, gummiartigen Zustand übergeht.

Der Ausdruck härtbare Dentalmassen umfasst alle Massen, die in Folge einer Polymerisationsreaktion, beispielsweise einer radikalisch, kationisch oder anionisch verlaufenden Additions- und/oder Kondensationsreaktion, und/oder Zementreaktion von einem viskosen, fließfähigen, ggf. plastisch verformbaren Zustand in einen dauerhaft verformten, festen Zustand übergehen können.

Massen, deren Abbindevorgang mit der erfindungsgemäßen Vorrichtung vorzugsweise erfassbar ist, weisen vor Beginn des Abbindevorgangs üblicherweise folgende Eigenschaften auf: Es handelt sich um pastöse, hochviskose Substanzen, die über einen Zeitraum im Bereich von 0,1 bis 60 min, vorzugsweise 1 bis 8 min erstarren. Solche Massen haben beispielsweise vor Beginn des Abbindevorgangs eine Viskosität bestimmt durch Konsistenzprüfung nach DIN 4823 von Klasse 0 bis 3, die mit einem Durchmesser von kleiner 80 mm gemessen werden. Als Materialien seien beispielhaft genannt Silikone, Polyether, Epoxide und Polyurethane.

Die Shore-Härte A der Massen gemessen nach DIN 53505 15 min nach Ende der Verarbeitungszeit liegt Üblicherweise im Bereich von 20 bis 110, vorzugsweise im Bereich von 30 und 80.

Insbesondere eignet sich die Erfindung zur Erfassung des Abbindeverhaltens von Dichtungs- und Dentalmassen, vorzugsweise dentalen Abformmassen, beispielsweise auf der Basis von Polyethern, A- und C-Silikonen, Alginaten und/oder Polyethersilikonen.

Unter rheologische Eigenschaft sind alle Eigenschaften zu verstehen, deren Veränderung über ein physikalisch und/oder chemisches Messverfahren erfasst werden kann. Hierunter fallen insbesondere Eigenschaften, wie Viskosität, Druckfestigkeit, pH-Wert, Leitfähigkeit, Dielektrizitätskonstante, Impedanz, Kapazität, Härte, Dichte und/oder Temperatur.

Der Begriff dynamischer Mischer umfasst im Gegensatz zu statischen Mischern, Mischer, die mindestens ein drehbar gelagertes Teil aufweisen, das über eine Mischerwelle angetrieben wird. Solche Mischer sind beispielsweise in der DE 90 17 323 U1 oder der WO-A-98/43727 beschrieben. Bei diesen Mischern handelt es sich um sog. Durchlaufmischer. Während des Mischens wird gemischtes Material bzw. Paste aus dem Mischer ausgebracht, wohingegen während des Messens das zumindest partiell gemischte Material im Mischer verbleibt und nicht weiter gefördert bzw. ausgebracht wird.

Ein dynamischer Mischer umfasst regelmäßig folgende Bestandteile: ein Gehäuse, Einlassöffnungen, eine Auslassöffnung und ein drehbar gelagertes Mischelement mit Mischflügeln. Der Antrieb erfolgt entweder mittig über einen Rotor mit daran befindlichen Mischflügeln, der sich im Gehäuse dreht, oder außermittig über einen als Rotor dienenden Teil des Gehäuses und eines als Stator dienenden Innenkörpers, wie es in der DE 19 947 331 A1 beschrieben wird.

Da es sich bei solchen Mischern üblicherweise um Wegwerfartikel handelt, deren Wert im Vergleich zu den zu mischenden Substanzen regelmäßig als gering zu bewerten ist, weisen derartige Mischer ein verhältnismäßig kleines Mischvolumen auf, um die Menge an zu verwerfender gemischter Masse möglichst gering zu halten. Derartige Einmalartikel werden üblicherweise aus Kunststoff gefertigt.

Eine Sensoreinheit im Sinne der Erfindung ist eine Einheit, die geeignet ist, irgendeine Zustandsänderung der Abformmasse über eine Kopplung an die Mischerwelle zu erfassen. Umfasst sind Kondensatoren, Ulraschallensoren. pH-Elektroden, Drehmomentaufnehmer, Schwingquarze, Thermoelemente, Strommesser, Spannungsmesser, Widerstandsmesser, Dehnungsmessstreifen und Kraftaufnehmer.

Unter den Begriff Anzeigeeinheit fallen alle Einheiten, die geeignet sind, den Benutzer der Vorrichtung über eine Zustandsänderung der Abformmasse zu informieren, vorzugsweise in optischer und/oder akustischer Form. Umfasst werden Displays, insbesondere mit LED-Anzeigen und Lautsprecher.

Mit den Begriffen "umfassen" oder "enthalten" wird eine nicht abschließende Aufzählung von Merkmalen eingeleitet. Der Umstand, dass in den Ansprüchen das Wort "ein" vor Nennung eines Merkmals verwendet wird, schließt nicht aus, dass die genannten Merkmale mehrmals vorhanden sein können, im Sinne von "mindestens ein".

Die Erfindung weist folgende Vorteile auf:

Die erfindungsgemäße Vorrichtung bzw. das erfindungsgemäße Verfahren ermöglichen das unmittelbare Verfolgen des Härtungsvorgangs der gemischten Masse, weitgehend unabhängig von äußeren Einflüssen.

Im Unterschied zu Messmischern, die aus dem Stand der Technik bekannt sind, erfolgt die Bestimmung der Veränderung der rheologischen Eigenschaften nicht an Hand einer Materialprobe, die im Anschluss daran weiter verwendet wird, sondern an Hand einer Materialprobe, die im Mischer verbleibt und zusammen mit diesem verworfen wird.

Die Messung kann direkt in der Vorrichtung erfolgen, die zum Mischen und Ausbringen der erhärtenden Dentalmassen üblicherweise verwendet wird. Das Anbringen zusätzlicher Hilfsmittel, wie Bypass, Kammern, Messkolben, Messantriebe oder Fühler an bekannte Mischvorrichtungen ist nicht notwendig.

Erfolgt die Messung ab dem Zeitpunkt, zu dem keine weitere gemischte Masse über den Mischer ausgebracht wird, ermöglicht die Erfindung die Erfassung des Härtungsvorgang der zuletzt gemischten Masse im Mischer selbst, der üblicherweise nach dem Aushärten der Masse verworfen wird. Insbesondere die Information, wann die zuletzt gemischte Masse auszuhärten beginnt, ist für den Anwender, beispielsweise den Zahnarzt bei der Anfertigung von Abformung des dentalen Hartgewebes, unter Verwendung von Abformmassen, von nicht zu unterschätzender Bedeutung.

Dies stellt einen entscheidenden Fortschritt gegenüber dem aus der DE 299 06 343 U1 bekannten Verfahren dar, bei dem die Zeit, bis zu der die Masse verarbeitet werden kann, voreingestellt und damit vom eigentlichen Abbindevorgang entkoppelt ist.

Die vorliegende Erfindung ermöglicht es somit, dynamische Mischer, die zum Mischen von erhärtenden Dentalmassen dienen nach dem Mischvorgang zur Verfolgung des Abbindeverlaufs der erhärtenden Dentalmassen zu verwenden. Dabei ist die Vorrichtung nicht auf einen bestimmten Mischertypus festgelegt. In Abhängigkeit von der zu mischenden Massen können unterschiedliche Mischer mit unterschiedlichen Geometrien verwendet werden. Das Auswechseln eines Mischers ist hierbei denkbar einfach und kann in weniger als einer Minute erfolgen.

Üblicherweise werden die Massen durch Mischen einer Basis- und einer Katalysatorpaste bereitet. Abhängig vom Mischungsverhältnis und den Substanzen erfolg der Abbindevorgang unterschiedlich schnell. Die erfindungsgemäße Vorrichtung eignet sich aber auch zur Bestimmung des Abbindevorgangs von einer Mischung aus drei oder mehreren Pasten oder Substanzen. Neben Paste/Paste-Mischungen kann es sich auch um Paste/Flüssigkeit- oder Pulver/Flüssigkeit-Mischungen handeln.

Das Mischen der Pasten erfolgt in einem dynamischen Mischer, beispielsweise gemäß der DE 90 17 323 U oder der WO-98/43727. Dieser wird üblicherweise auf eine entsprechende Kartusche aufgesteckt und mit einem elektrisch betriebenen Mischgerät, in das die Kartusche eingelegt werden kann, angetrieben. Geeignete Mischgeräte sind in der EP 0 492 413 A beschrieben.

Handelt es sich bei der durch Mischen erhaltenen Dentalmasse um eine dentale Abformmasse, wird mit dieser vorzugsweise ein dentaler Abformlöffel befüllt, der anschließend in den Mundraum eines Patienten eingesetzt wird.

Da in diesem Fall der Abbindeverlauf gemäß der Erfindung außerhalb des Mundraumes gemessen wird und der Verlauf temperaturabhängig ist, erfolgt die Abbindung der Masse im Gerät etwas langsamer als im Mundraum. Dadurch ist sichergestellt, dass die Masse in jedem Fall vollständig abgebunden hat, bevor sie aus dem Mundraum entnommen wird.

Die direkte Korrelation zwischen Abbindeverlauf im Mundraum und im Mischgerät lässt sich empirisch in Abhängigkeit von der verwendeten Masse ermitteln.

Die Messung der Änderung einer rheologischen Eigenschaft der erhärtenden Dentalmasse und der Abgleich mit einem vorgebbarem Schwellenwert erfolgt über ein Messprogramm, das in die Steuerung der Mischerwelle integriert sein kann oder unabhängig von dieser gefahren wird.

Vorzugsweise erfolgt die Messung unmittelbar über die Mischerwelle eines elektrisch betriebenen Mischgeräts, die einen dynamischen Mischer antreibt. Nach dem Befüllen eines Abformlöffels wird die Mischerwelle bei abgeschaltetem Vorschub weiterhin angetrieben, wobei mit zunehmender Verfestigung der Masse das an der Welle anliegende Drehmoment zunimmt. Das Ende der Verarbeitungszeit der Masse kann in dieser Ausführungsform durch ein optisches oder akustisches Signal angezeigt werden, sobald ein bestimmter Schwellenwert erreicht wird, der je nach Masse individuell einstellbar ist bzw. automatisch eingestellt wird, sobald die Kartusche, die die Masse enthält in das Mischgerät eingelegt wird.

Die Mischerwelle ist beispielsweise mit einem Drehmomentaufnehmer oder einer verdrehbaren Welle gekoppelt, welche es ermöglichen, das an den Mischerflügeln des Mischers bzw. an der Mischerwelle anliegende Drehmoment zu bestimmen, welches proportional zur Viskosität der erhärtenden Dentalmasse ist. Möglich ist auch, die Veränderung des Drehmoments über die Stromaufnahme des Antriebs zu erfassen. Vorzugsweise erfolgt die Messung der Änderung mit einer im Vergleich zum Mischen reduzierten Drehzahl der Mischerwelle, um nicht unnötig Rotationsenergie in die erhärtende Masse einzubringen, was zu einer unerwünschten Temperaturerhöhung führen kann. Dies erlaubt zudem eine genauere Bestimmung der Änderung. Brauchbare Drehzahlen für die Mischerwelle während des Messvorgangs liegen im Bereich von 10° bis 10³ vorzugsweise im Bereich 10¹ bis 10² U/min.

In einer alternativen Ausführungsform erfolgt der Messvorgang über die Mischerwelle in einem Pulsbetrieb oder getaktet. Beispielsweise erfolgt die Messung nur alle 1, 5 oder 10 sec.

Die Messung der Änderung einer rheologischen Eigenschaft der härtenden Masse kann auch erfolgen, wenn die Mischerwelle nicht vollständig rotiert. Eine Drehung der Mischerwelle in einem Winkelbereich von 1 bis 180°, vorzugsweise 10 bis 90° hat sich als vorteilhaft erwiesen.

Als Messeinheit eignet sich aber beispielsweise auch ein Schwingquarz, der eine reine Torsionsschwingung erzeugt, die durch die viskosen Eigenschaften der erhärtenden Masse gedämpft wird. Der Sensorkopf kann in Form eines Fühlers an oder im Bereich der Spitze der Mischerwelle angebracht sein und über eine Dichtung im Bereich der Kupplung dynamischer Mischer/Mischerwelle in die Masse eingeführt werden.

Im Unterschied zu aus dem Stand der Technik bekannten Messmischem, kann mit der vorliegenden Vorrichtung nicht nur eine härtbare Masse gemischt und gefördert werden, sondern auch im Anschluss an den Misch- und Förderprozess der Abbindeverlauf des Teils der gemischten Masse, der im auswechselbaren, verwerfbaren Mischer verbleibt, bestimmt werden.

Ein bevorzugtes Ausführungsbeispiel wird nachstehend anhand einer Figur erläutert.

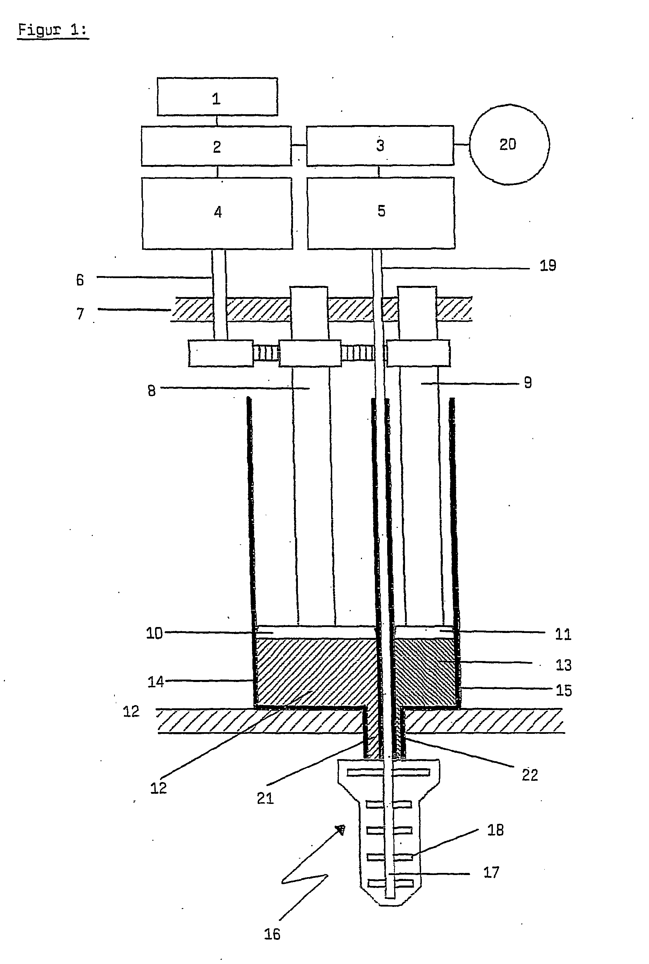

- Figur 1: zeigt schematisch eine mögliche Anordnung der einzelnen Bestandteile der Messvorrichtung.

Durch Drücken des Förderungsschalters (1) wird über die Hubkolbensteuerung (2) der Kolbenmotor mit einer bestimmten Drehzahl (4) aktiviert, der über die Antriebswelle (6) und ein Antriebselement (7), beispielsweise ein Zahnriemen, die Förderspindeln (8, 9) und die zugehörigen Kolben (10, 11) in axiale Richtung vorwärtsbewegt.

Die Kolben (10, 11) werden in Kartuschen (14, 15) geführt, die die einzelnen Komponenten der härtenden Masse enthalten. Durch die axiale Bewegung der Kolben werden die beiden Komponenten über die Auslassöfifnungen (21, 22) der Kartuschen in einen dynamischen Mischer (16) gedrückt.

Gleichzeitig mit der Aktivierung der Hubkolbensteuerung (2) erfolgt die Aktivierung der Mischerwellensteuerung (3), um den Mischerwellenmotor mit der für den Mischvorgang notwendigen Drehzahl zu beaufschlagen, die über die Mischerwelle (19) auf die Mischerwendel (17) des dynamischen Mischers (16) übertragen wird. Die Aktivierung der Mischerwellensteuerung kann über die Hubkolbensteuerung oder unabhängig von dieser erfolgen. Die rotierenden Mischflügel (18) sorgen für eine intensive und homogene Mischung der beiden strömenden, pastösen Komponenten (12, 13), so dass eine direkte Applikation beispielsweise in einen hier nicht gezeigten Abformlöffel erfolgen kann.

Nach vollständiger Befüllung des Abformlöffels wird der Förderungsschalter (1) losgelassen, wodurch die Kolbenhubsteuerung (2) den Förderbetrieb einstellt. In diesem Moment schaltet die Mischerwellensteuerung (3) auf Messen um. D.h., nach beispielsweise 10 sec. Stillstand des Mischerwellenmotors (5) wird die Mischerwelle (19) in einer für die Torsionsmessung langsameren und konstant definierten Drehzahl um beispielsweise 180° gedreht und der hiefür benötigte Strom I gemessen.

Sobald die im Mischer (16) befindliche Masse zu erhärten beginnt, erhöht sich proportional dazu die Stromstärke I, die benötigt wird, um die Mischerwelle auf konstanter Drehzahl zu halten. Ab einem bestimmten Zustand der härtenden Masse wird ein kritischer, in der Vorrichtung für die jeweilige Masse gespeicherter oder eingebbarer Schwellenwert für die Stromstärke I überschritten, so dass die Mischerwellensteuerung (3) die Anzeigeeinheit (20) aktiviert, die daraufhin ein akustisches oder optisches Signal abgibt.

### Bezugszeichenliste

1. Pastenförderungsschalter
2. Hubkolbensteuerung
3. Mischerwellensteuerung
4. Kolbenhubmotor
5. Mischerwellenmotor
6. Antriebswelle
7. Antriebselement
8. Förderspindel
9. Förderspindel
10. Kolben
11. Kolben
12. Komponente A
13. Komponente B
14. Kartusche A
15. Kartusche B
16. Dynamischer Mischer
17. Mischerwendel
18. Mischflügel
19. Mischerwelle
20.Anzeigeeinheit
21.Auslassöffnung A
22. Auslassöffnung B

## Patentansprüche

1. Vorrichtung zur Verfolgung des Abbindeverlaufs von erhärtenden Dentalmassen, umfassend einen Mischerwellenmotor mit einer Steuerung, eine Mischerwelle, eine Aufnahme für eine Kartusche, die mit einem dynamischen Einweg-Mischer verbindbar ist, welcher zum Mischen der Dentalmasse dient, eine Anzeigeeinheit und eine Sensoreinheit, die die Änderung mindestens einer der rheologischen Eigenschaften der Dentalmasse erfassen kann, wobei die Sensoreinheit mit dem Mischerwellenmotor gekoppelt ist und der Einweg-Mischer mit der Sensoreinheit zusammenwirkt, wobei die rheologischen Eigenschaften gewählt sind aus Viskosität, Druckfestigkeit, pH-Wert, Leitfähigkeit, Dielektrizitätskonstante, Impedanz, Kapazität, Härte, Dichte und/oder Temperatur. Vorrichtung nach Ansprüch 1, wobei die Sensoreinheit

2. gewählt ist aus: Kondensatoren, Ultraschallsensoren, pH-Elektroden, Drehmomentaufnehmer, Schwingquarze, Thermoelemente, Strommesser, Spannungsmesser, Widerstandsmesser, Dehnungsmessstreifen und/oder Kraftaufnehmer.

3. Vorrichtung nach einem der vorstehenden Ansprüche, wobei die Anzeigeeinheit ein optisches und/oder akustisches Signal abgeben kann.

4. Vorrichtung nach einem der vorstehenden Ansprüche, wobei die Sensoreinheit die Änderung des Drehmoments erfassen kann, das an der Mischerwelle anliegt.

5. Verfahren, umfassend die Schritte: a) Mischen einer erhärtenden Dentalmasse in einem dynamischen Einweg-Mischer, der von einem Mischerwellenmotor angetrieben wird, b) weitgehend vollständiges Ausbringen der Dentalmasse aus dem dynamischen Einweg-Mischer, c) Erfassung der Änderung mindestens einer der rheologischen Eigenschaften der gemischten Dentalmasse, die im dynamischen Einweg-Mischer verbleibt, über eine Sensoreinheit, d) Abgabe eines Signals, sobald der Wert der Änderung aus Schritt c) einen voreinstellbaren Schwellenwert erreicht hat, wobei die Sensoreinheit mit dem Mischerwellenmotor zusammenwirkt , wobei zumindest Schritt c) mit einer Vorrichtung nach einem der Ansprüche 1 bis 4 durchgeführt wird.

6. Verfahren nach Ansprüch 5 , wobei in Schritt c) eine Änderung des Drehmoments erfasst werden kann, das an der Mischerwelle anliegt.

7. Verfahren nach einem der Ansprüche 5 bis 6, wobei die Messung in Schritt c) gepulst erfolgt.

8. Verfahren nach einem der Ansprüche 5 bis 7, wobei die Messung in Schritt c) mit einer Drehzahl erfolgt, die kleiner ist als, die Drehzahl, die in Schritt a) zum Mischen verwendet wird.

9. Verfahren nach einem der Ansprüche 5 bis 8, wobei während der Messung in Schritt c) die Mischerwelle nicht vollständig rotiert.

10. Verwendung einer Vorrichtung nach einem der Ansprüche 1 bis 4 oder des Verfahrens nach einem der Ansprüche 5 bis 9 zur Bestimmung des Endes der Verarbeitungszeit von erhärtenden Dentalmassen.

11. Verwendung nach Anspruch 10, wobei es sich bei der erhärtenden Masse um eine dentale Abformmasse handelt.

12. Verwendung eines dynamischen Einweg-Mischers gemäß der Vorrichtung nach einem der vorstehenden Ansprüche 1-4 zum Mischen, Ausbringen und im Anschluss daran Verfolgen des Abbindeverlaufs von erhärtenden dentalmassen.

## Claims

1. Device for monitoring the setting profile of hardening dental compounds, comprising a mixer-shaft motor with a control, a mixer shaft, a seat for a cartridge, which can be connected to a dynamic disposable mixer which is used to mix the hardening dental compound, a display unit and a sensor unit, which can record the change in at least one of the rheological properties of the dental compound, the sensor unit being coupled to the mixer-shaft motor, and the disposable mixer cooperating with the sensor unit,
in which the rheological properties are chosen from viscosity, compressive strength, pH, conductivity, dielectric constant, impendance, capacitance, hardness, density and/or temperature.

2. Device according to Claim 1, in which the sensor unit is chosen from: capacitors, ultrasound sensors, pH electrodes, torque transducers, quartz oscillators, thermoelements, current meters, voltage meters, resistance meters, wire strain gages and/or force transducers.

3. Device according to one of the preceding claims, in which the display unit can output a visual and/or acoustic signal.

4. Device according to one of the preceding claims, in which the sensor unit can record the change in the torque applied to the mixer shaft.

5. Method comprising the following steps: a) mixing a hardening dental compound in a dynamic disposable mixer which is driven by a mixer-shaft motor, b) dispensing almost all of the dental compound from the dynamic disposable mixer, c) recording the change in at least one of the rheological properties of the mixed dental compound which remains in the dynamic disposable mixer via a sensor unit, d) outputting a signal as soon as the value of the change from step c) has reached a predeterminable threshold value, the sensor unit cooperating with the mixer-shaft motor, in which at least step c) is performed with a device according to one of Claims 1 to 4.

6. Method according to Claim 5, in which, in step c), a change in the torque applied to the mixer shaft can be recorded.

7. Method according to one of Claims 5 to 6, in which the measurement in step c) is pulsed.

8. Method according to one of Claims 5 to 7, in which the measurement in step c) takes place with a speed of rotation which is smaller than the speed of rotation which is used in step a) for mixing.

9. Method according to one of Claims 5 to 8, in which the mixer shaft is not fully rotated during the measurement in step c).

10. Use of the device according to one of Claims 1 to 4 or of the method according to one of Claims 5 to 9 for determining the end of the processing time of hardening dental compounds.

11. Use according to Claim 10, in which the hardening compound is a dental impression compound.

12. Use of a dynamic disposable mixer according to the device according to one of the preceding Claims 1-4 for mixing, dispensing and subsequently monitoring the setting profile of hardening dental compounds.

## Revendications

1. Dispositif pour suivre le déroulement de la prise de masses dentaires durcissables, comprenant un moteur d'arbre de mélangeur doté d'une commande, un arbre de mélangeur, un logement pour une cartouche qui peut être relié avec un mélangeur dynamique à usage unique, lequel sert à mélanger la masse dentaire durcissable, une unité d'indication et une unité de détection qui peut détecter la modification d'au moins l'une des propriétés rhéologiques de la masse dentaire, l'unité de détection étant couplée avec le moteur d'arbre de mélangeur et le mélangeur à usage unique interagissant avec l'unité de détection, les propriétés rhéologiques étant choisies dans le groupe composé de la viscosité, la résistance à la compression, la valeur pH, la conductivité, la constante diélectrique, l'impédance, la capacité, la dureté, la densité et/ou la température.

2. Dispositif selon la revendication 1, l'unité de détection étant choisie parmi le groupe composé de condensateurs, capteurs à ultrasons, électrodes de pH, détecteurs de couple, quartz oscillants, éléments thermostatiques, mesureurs de courant, mesureurs de tension, mesureurs de résistance, jauges extensométriques et/ou capteurs dynamométriques.

3. Dispositif selon l'une des revendications précédentes, l'unité d'indication pouvant délivrer un signal visuel et/ou sonore.

4. Dispositif selon l'une des revendications précédentes, l'unité de détection pouvant détecter la modification du couple qui est appliqué à l'arbre du mélangeur.

5. Procédé comprenant les étapes suivantes : a) Mélange d'une masse dentaire durcissable dans un mélangeur dynamique à usage unique qui est entraîné par un moteur d'arbre de mélangeur, b) prélèvement quasi-total de la masse dentaire hors du mélangeur dynamique à usage unique, c) détection, par le biais d'une unité de détection, de la modification d'au moins l'une des propriétés rhéologiques de la masse dentaire mélangée qui demeure dans le mélangeur dynamique à usage unique, d) délivrance d'un signal dès que la valeur de la modification de l'étape c) a atteint une valeur de seuil pouvant être préréglée, l'unité de détection interagissant avec le moteur d'arbre de mélangeur, au moins l'étape c) étant exécutée avec un dispositif selon l'une des revendications 1 à 4.

6. Procédé selon la revendication 5, une modification du couple qui est appliqué à l'arbre du mélangeur pouvant être détectée à l'étape c).

7. Procédé selon l'une des revendications 5 à 6, la mesure à l'étape c) étant effectuée de manière impulsionnelle.

8. Procédé selon l'une des revendications 5 à 7, la mesure à l'étape c) étant effectuée à une vitesse de rotation qui est inférieure à la vitesse de rotation qui est utilisée à l'étape a) pour le mélange.

9. Procédé selon l'une des revendications 5 à 8, l'arbre de mélangeur n'effectuant pas une rotation complète pendant la mesure à l'étape c).

10. Utilisation d'un dispositif selon l'une des revendications 1 à 4 ou du procédé selon l'une des revendications 5 à 9 pour déterminer la fin du temps de traitement de masses dentaires durcissables.

11. Utilisation selon la revendication 10, la masse durcissable étant une masse de moulage dentaire.

12. Utilisation d'un mélangeur dynamique à usage unique conformément au dispositif selon l'une des revendications précédentes 1 à 4 pour mélanger, prélever et ensuite suivre le déroulement de la prise de masses dentaires durcissables.
